Europäisches Patentamt

European Patent Office

Office européen des brevets

(19)

(11) Numéro de publication : **0 283 349 B1**

(12) **FASCICULE DE BREVET EUROPEEN**

(45) Date de publication du fascicule du brevet :
25.03.92 Bulletin 92/13

(51) Int. Cl.⁵ : **A61K 35/78, A61K 7/48**

(21) Numéro de dépôt : **88400382.3**

(22) Date de dépôt : **19.02.88**

(54) **Procédé d'obtention de principes tanniques d'origine végétale, composition cosmétologique et composition pharmaceutique contenant le principe tannique purifié obtenu selon le procédé.**

Le dossier contient des informations
techniques présentées postérieurement au
dépôt de la demande et ne figurant pas dans le
présent fascicule.

(30) Priorité : **20.02.87 FR 8702229**

(43) Date de publication de la demande :
**21.09.88 Bulletin 88/38**

(45) Mention de la délivrance du brevet :
**25.03.92 Bulletin 92/13**

(84) Etats contractants désignés :
**AT BE CH DE ES FR GB GR IT LI LU NL SE**

(56) Documents cités :
**CHEMICAL ABSTRACTS, vol. 81, no. 10, 9
septembre 1974, page 317, résumé no. 54372p,
Columbus, Ohio, US; N.N. NOVIKOVA:
"Application of Filipendula ulmaria for some
therapeutic preparations", & NAUCH. TR.,
PERM. FARM. INST. 1973, 5(2), 27-30**

(56) Documents cités :
**CHEMICAL ABSTRACTS, vol. 88, no. 3, 16
janvier 1978, page 318, résumé no. 19061j,
Columbus, Ohio, US; O.D. BARNAULOV et al.:
"Chemical composition and primary evaluation of the properties of preparations from
Filipendula ulmaria (L.) Maxim flowers", &
RASTIT. RESUR. 1977, 13(4), 661-9
CHEMICAL ABSTRACTS, vol. 75, no. 6, 9 août
1971, page 275, résumé no. 40301d, Columbus,
Ohio, US; N.N. NOVIKROVA: "Use of Filipendula ulmaria in medicine", & TR. PERM.
FARM. INST. 1969, no. 3, 267-70**

(73) Titulaire : **Lamaison, Jean-Louis
21 bis, rue de Champiot
F-63830 Durtol (FR)**

(72) Inventeur : **Lamaison, Jean-Louis
21 bis, rue de Champiot
F-63830 Durtol (FR)**

(74) Mandataire : **Warcoin, Jacques et al
Cabinet Régimbeau 26, avenue Kléber
F-75116 Paris (FR)**

EP 0 283 349 B1

Jouve, 18, rue Saint-Denis, 75001 PARIS

## Description

La présente invention concerne un procédé d'obtention de principes tanniques d'origine végétale purifiés, ainsi que l'application en cosmétologie et en pharmacologie des principes actifs obtenus par ledit procédé.

Plus précisément, le but de la présente invention est d'obtenir, à partir de matières premières d'origine végétale, les principes tanniques actifs sous forme d'extraits purifiés de manière pratiquement quantitative, les extraits purifiés renfermant ainsi la presque totalité des activités anti-enzymes d'origine.

Pour ce faire, la présente invention a pour objet un procédé d'obtention de principes tanniques purifiés d'origine végétale qui, selon sa caractéristique essentielle, comporte les étapes suivantes :

a) l'adsorption du principe tannique actif à partir d'une solution extractive de la matière première sur un adsorbant polymère à fonctions amides ou équivalentes,

b) la désorption sélective et quantitative du principe tannique actif fixé sur l'adsorbant, désorption à l'aide de bases ou de substances à propriétés basiques.

Il convient de remarquer qu'il était communément admis avant la présente invention que les tannins en général étaient dégradés, notamment par oxydation par les substances basiques et que plus particulièrement les tannins ellagiques pouvaient être saponifiés facilement en milieu alcalin avec formation de sels d'acides correspondants et donc que leur emploi comme solvants de désorption était à proscrire. Or de manière surprenante, ils se sont avérés, selon la présente invention, particulièrement appropriés pour la désorption des tannins sur polymères polyamides.

Dans un mode de réalisation particulièrement avantageux, la solution peut ensuite être neutralisée par addition d'acide ou de substances à propriétés acides de quelconque nature, pures ou en mélanges.

Avantageusement, entre l'étape d'adsorption a) et l'étape de désorption b) des substances banales de la plante sont éliminées quantitativement par lavage ou élution à l'aide de solvants.

Ces substances banales de la plante sont notamment des sels minéraux, sucres, protides, certaines substances phénoliques telles qu'acide-phénols, phénols et dérivés, flavones, etc.

De manière particulièrement avantageuse, la plante d'origine est la Reine des Prés - Filipendula Ulmaria (Flora Europaea) ou Spiraea Ulmaria - de la famille des Rosacées, l'espèce proprement dite, ses sous-espèces, ses variétés ou espèces affines, notamment d'origine horticole et les hybrides éventuels.

Les raisons du choix de cette matière première s'explique d'après les résultats très intéressants obtenus comparativement à ceux d'autres plantes ou principes actifs d'origine végétale - certains présents dans la Reine des Prés comme l'acide gallique, l'acide chlorogénique, le salicylate de méthyle, le rutoside, l'hypéroside, la quercétine-4' glucoside, principale flavone des fleurs, la quercétine et le principe tannique - et reproduits dans le tableau figurant à l'exemple 6.

La matière première précitée présente des activités biochimiques en tant qu'anti-enzymes et des activités physiologiques résultantes protectrices cellulaires et vasculaires.

Elle inhibe notamment diverses enzymes protéolytiques telles que l'élastase, la collagénase et l'hyaluronidase qui participent à la dégradation des trames des organites cellulaires, des membranes, des tissus tels que le tissu conjonctif par exemple ou des parois tissulaires.

Parmi les polymères d'adsorption à fonctions amides ou équivalentes, on peut citer particulièrement les nylons®, les polyamides, les polyvinylpyrrolidones, soit purs, soit mélangés entre eux ou dilués.

Avantageusement, la désorption se fait avec des bases ou des substances à propriétés basiques, fortes ou faibles, en solutions aqueuses, alcooliques, ou cétoniques pures ou en mélanges.

Par exemple, la substance à propriétés basiques réalisant la désorption est une solution d'hydroxyde de sodium ou une solution d'ammoniaque.

Lorsque le produit actif est désorbé à l'aide de bases ou de substances à propriétés basiques en solution, le titre de la solution est choisi de manière à ce que la désorption soit quantitative.

La neutralisation de la solution peut se faire par addition d'acides ou substances à propriétés acides, par utilisation de résines échangeuses d'ions, par dialyse ou tout autre procédé de séparation moléculaire.

A l'origine, la drogue fraîche ou desséchée selon un quelconque procédé peut être divisée de manière suffisante sous forme de poudre afin de faciliter l'extraction.

L'extraction de la matière première une fois traitée est réalisée à l'aide de solvants et effectuée suivant un procédé à chaud ou à froid à l'aide d'un appareillage d'extraction de quelconque nature opérant en continu ou en discontinu de manière à ce que l'extraction soit quantatitive.

Une extraction préliminaire de manière à éliminer des substances banales gênant l'extraction peut être réalisée par exemple avec un solvant de polarité différente de celui de l'extraction.

Après extraction, les parties végétales peuvent être séparées de la solution extractive à l'aide d'un appareillage adapté par exemple de filtration ou de centrifugation.

La solution extractive précitée peut être concentrée ou évaporée à siccité et reprise par des solvants.

Parmi les solvants, notamment d'extraction ou de lavage, on peut citer :

. les solvants hydroxylés par exemple l'eau, les solutions aqueuses salines, acides ou basiques, les alcools, notamment le méthanol, l'éthanol, le propanol ou encore le propylèneglycol, le glycérol,

. les solvants cétoniques, notamment l'acétone, le méthyléthylcétone,

. les esters tels que acétate d'éthyle, acétate d'isopropyle,

. ou tout autre solvant à caractère relativement polaire tels que le tétrahydrofuranne.

Ces solvants peuvent être utilisés soit purs, soit mélangés entre eux en quelconques proportions.

Comme on l'a vu, la solution de désorption peut être neutralisée et les liqueurs de désorption ainsi obtenues peuvent être ensuite traitées de manière à obtenir le produit actif sous forme sèche. Elles peuvent être concentrées de manière telle que le produit actif précipite de la solution, la précipitation étant rendue quantitative par exemple par refroidissement ou tout autre procédé permettant de quantifier cette opération.

Le produit actif peut être récupéré suivant une opération de séparation telle que par exemple une centrifugation et une filtration ou tout autre type d'opérations permettant l'élimination du liquide et la récupération du produit actif sous forme solide.

Le principe actif peut alors être soumis à une opération de dessiccation de quelconque nature de manière à obtenir un produit suffisamment sec pour pouvoir être réduit en poudre.

Le principe actif peut ainsi être utilisé sous forme sèche ou sous forme liquide, par exemple en solution dans des solvants tels que ceux précités.

Dans un mode de réalisation particulier, la solution extractive est extraite de poudre de fleurs de Reine des Prés. Puis elle est filtrée, puis concentrée jusqu'à élimination du solvant. Le résidu est ensuite repris avec de l'eau, soumis à une température supérieure à la température ambiante de manière à faciliter la dissolution. La solution aqueuse obtenue est déposée en tête d'une colonne contenant de la poudre de polymère polyamide.Après adsorption de la solution aqueuse la colonne est lavée avec un solvant, la matière banale étant ainsi éliminée. L'élution est ensuite réalisée à l'aide de solutions hydroalcooliques et d'hydroxyde de sodium ou d'ammoniaque, l'éluat étant neutralisé en cours d'élution avec de l'acide chlorhydrique ou de l'acide acétique. La solution est ensuite soumise à la dialyse, puis la liqueur de dialyse est concentrée jusqu'à apparition d'un précipité par exemple à l'aide d'un évaporateur rotatif sous pression réduite. La liqueur concentrée est ensuite réfrigérée à 4°C de manière à quantifier la précipitation. La liqueur est filtrée sur un Büchner. Le précipité est lavé et séché. Le produit sec est alors pulvérisé.

L'invention a également pour objet les applications de ce principe actif utilisant leurs activités anti-enzymatiques.

L'invention a en outre pour objet des compositions cosmétologiques et des compositions pharmaceutiques comportant à titre de principe actif les principes tanniques purifiés obtenus selon le présent procédé.

D'autres caractéristiques et avantages de la présente invention apparaîtront à la lumière de la description présentée sous forme d'exemples.

## EXEMPLE 1 - ORIGINE DE LA MATIERE PREMIERE

La plante d'origine est la Reine des Prés - Filipendula Ulmaria (Flora Europaea) = Spiraea Ulmaria - de la famille des Rosacées, l'espèce proprement dite, ses sous-espèces, ses variétés ou espèces affines, notamment d'origine horticole et les hybrides éventuels.

La plante très cosmopolite peut être à l'origine soit sauvage, soit cultivée.

La matière première - ou drogue - est constituée par les fleurs ou parties de fleurs, les sommités fleuries ou tout autre partie végétale de composition analogue.

## EXEMPLES 2 A 4 - PREPARATION DE L'EXTRAIT ACTIF

### Exemple 2

1 kg de poudre de fleurs de Reine des Prés (tamis 250) sont extraits à chaud dans un appareil d'extraction en continu de type SOXHLET à l'aide de 15 litres d'éthanol à 80 pour cent V/V pendant 48 heures jusqu'à épuisement de la poudre.

Après refroidissement, la liqueur extractive est filtrée sur papier.

La solution extractive est concentrée jusqu'à consistance semi-pâteuse en opérant à l'aide d'un évaporateur rotatif sous pression réduite.

Le résidu est repris avec 2 litres d'eau injectés directement dans le ballon d'évaporation agité par rotation et soumis à une température de 50°C pendant 10 minutes, de manière à faciliter la dissolution.

La solution aqueuse est déposée en tête d'une colonne de 15 cm de diamètre et de 100 cm de hauteur, contenant 1 kg de poudre de polyamide préalablement lavée avec 10 litres d'éthanol à 96c puis avec 10 litres d'eau.

Après adsorption de la solution aqueuse, la colonne est lavée avec 20 litres d'eau puis avec 20 litres d'éthanol à 96c, les matières banales étant ainsi éliminées.

L'élution est réalisée à l'aide de 20 litres de solution hydroalcoolique à 50 pour cent V/V d'hydroxyde de sodium 0,2 N, l'éluat étant neutralisé en cours d'élution avec de l'acide chlorhydrique 1 N en utilisant un titrimètre automatique.

La solution est introduite dans des boudins à dialyse et soumise à une dialyse pendant 48 heures dans un bac à circulation d'eau à 12°C.

La liqueur de dialyse est concentrée à l'aide d'un évaporateur rotatif sous pression réduite jusqu'à apparition d'un précipité pour un volume d'environ un litre.

La liqueur concentrée est réfigérée à + 4°C pendant une nuit, de manière à quantifier la précipitation.

La liqueur est filtrée sur Büchner à l'aide d'une fiole à vide de manière à bien essorer le précipité.

Le précipité est séché dans un étuve à soufflerie à une température de 70°C pendant 24 heures.

Le produit sec est alors pulvérisé dans un broyeur à couteaux de manière à obtenir une poudre passant au tamis module 250.

On obtient ainsi 40 grammes de poudre constituant le principe actif, tannin de nature probablement ellagique.

Exemple 3

1 kg de poudre de fleurs de Reine des Prés (tamis 250) sont extraits a chaud dans un appareil d'extraction en continu de type SOXHLET à l'aide de 15 litres d'éthanol à 80 pour cent V/V pendant 48 heures jusqu'à épuisement de la poudre.

Aprés refroidissement, la liqueur extractive est filtrée sur papier.

La solution extractive est concentrée jusqu'à élimination de l'alcool à l'aide d'un évaporateur sous pression réduite.

Le résidu est repris avec 2 litres d'eau injectés directement dans le ballon d'évaporation agité par rotation et soumis à une température de 50°C pendant 10 minutes, de manière à faciliter la dissolution.

La solution aqueuse est déposée en tête d'une colonne de 15 cm de diamètre et de 100 cm de hauteur, contenant 1 kg de poudre de Nylon® préalablement lavée avec 10 litres d'éthanol à 96c puis avec 10 litres d'eau.

Après adsorption de la solution aqueuse, la colonne est lavée avec 20 litres d'eau, puis avec 20 litres d'éthanol à 96c, les matières banales étant ainsi éliminées.

L'élution est réalisée à l'aide de 5 litres de solution hydroalcoolique à 50 % V/V d'ammoniaque 2 N, l'éluat étant neutralisé en cours d'élution avec de l'acide acétique en utilisant un titrimètre automatique et un récipient réfrigéré.

La solution est concentrée à l'aide d'un évaporateur rotatif sous pression réduite jusqu'à précipitation, soit un volume d'environ un litre.

La liqueur concentrée est réfrigérée à + 4°C pendant une nuit, de manière à ce que la précipitation soit quantitative.

La liqueur est filtrée sur Büchner à l'aide d'une fiole à vide de manière à bien essorer le précipité.

Le précipité est lavé directement sur le filtre avec deux fois 100 ml d'eau, puis essoré.

Le précipité est séché dans une étuve à soufflerie à une température de 70°C environ pendant 24 heures.

Le produit sec est alors pulvérisé dans un broyeur à couteaux de manière à obtenir une poudre passant au tamis module 250.

On obtient ainsi 36 grammes de poudre constituant le principe actif.

Exemple 4

1 kg de poudre de fleurs de Reine des Prés (tamis 250) sont extraits à chaud dans un appareil d'extraction en continu de type SOXHLET à l'aide de 15 litres d'éthanol à 80 pour cent V/V pendant 48 heures jusqu'à épuisement de la poudre.

Après refroidissement, la liqueur extractive est filtrée sur papier.

La solution extractive est concentrée jusqu'à élimination de l'alcool à l'aide d'un évaporateur sous pression réduite.

Le résidu est repris avec 2 litres d'eau injectés directement dans le ballon d'évaporation agité par rotation et soumis à une température de 50°C pendant 10 minutes, de manière à faciliter la dissolution.

La solution est déposée en tête d'une colonne de 15 cm de diamètre et de 100 cm de hauteur, contenant 1 kg de poudre de polyamide préalablement lavée avec 10 litres d'éthanol à 96c, puis avec 10 litres d'eau.

Après adsorption de la solution aqueuse, la colonne est lavée avec 20 litres d'eau, puis avec 20 litres d'éthanol à 96c, les matières banales étant ainsi éliminées.

L'élution est réalisée à l'aide de 2,5 litres de solution hydroalcoolique à 50 pour cent V/V d'hydroxyde de sodium 2 N, l'éluat étant neutralisé en cours d'élution avec de l'acide chlorhydrique en utilisant un titrimètre automatique et un récipient réfrigéré.

La solution est concentrée à l'aide d'un évaporateur rotatif sous pression réduite jusqu'à un volume d'environ un litre.

La liqueur est filtrée sur Büchner à l'aide d'une fiole à vide de manière à bien essorer le précipité, après réfrigération à 4°C pendant une nuit.

Le précipité est lavé directement sur le filtre avec deux fois 100 ml d'eau, puis essoré.

Le précipité est séché dans une étuve à soufflerie à une température ne dépassant pas 70°C pendant 24 heures.

Le produit sec est alors pulvérisé dans un broyeur à couteaux de manière à obtenir une poudre passant au tamis module 250.

On obtient ainsi 33 grammes de poudre constituant le principe actif.

EXEMPLE 5 - CONTROLE DU PRINCIPE ACTIF

Ci-après sont décrits les caractères du principe actif lorsqu'il est obtenu sous forme sèche en poudre.

5 - 1 - Composition :

Le principe actif est constitué par les tannins actifs purifiés de la plante d'origine, à l'exclusion de tout autre constituant en quantité notable.

La teneur en tannin par rapport au poids sec est au moins égale à 95 pour cent.

5 - 2 - Caractères et solubilités :

Poudre de couleur gris brun pratiquement inodore, de saveur astringente, peu soluble dans l'eau, plus soluble dans les solutions acides ou basiques, hydroalcooliques ou hydrocétoniques, pratiquement insoluble dans les solvants organiques tels que l'éther, les solvants chlorés, etc.

5 -3 - Identification :

Le produit présente les réactions colorées générales des tannins telles que sels ferriques, bleu solide, etc., et se fixe sur les supports polyamidés, précipite la gélatine en solution, etc.

Il présente des valeurs maximales dans l'U.V. aux longueurs d'onde voisines de 275 nm et de 225 nm, comme par exemple dans l'éthanol à 50 % V/V.

Dans l'infrarouge, pastillé dans le KBr, il présente des signaux caractéristiques des hydroxyles au-delà de 3 000 cm$^{-1}$, des fonctions esters à environ 1 700 cm$^{-1}$, des noyaux aromatiques à environ 1 500 cm$^{-1}$ qui sont les bandes principales.

Le principe actif peut être caractérisé par CCM, dans les conditions suivantes par exemple :
– support : gel de silice G - solvant de migration : acétate d'éthyle - acide formique - eau : 5 : 3 : 1 V/V/V - réactif de révélation : UV à 254 nm - dépôt : 5 μl de solution à 0,5 pour cent m/V dans l'éthanol à 50 pour cent V/V - distance de migration : 10 cm - résultat : taches foncées de Rf voisins de 0,50.

5 - 4 - Essai :

. Teneur en eau : déterminée selon la méthode CODEX de perte à la dessiccation, à l'étuve à 100-105°C, la teneur en eau "h" ne devra pas être supérieure à 10 pour cent.

. Taux de matières minérales : déterminé selon la méthode CODEX des cendres totales, le taux de matières minérales ne devra pas être supérieur à 0,5 pour cent.

5 - 5 - Dosage des tannins

Déterminé selon la méthode d'adsorption sur la poudre de peau, le titre en tannin devra être au moins égal à 95 pour cent, rapporté au produit sec.

On peut opérer par exemple selon le mode suivant.

On agite mécaniquement pendant 30 minutes 250 ml de solution à 0,5 pour cent m/V dans l'éthanol à 50 pour cent V/V avec 10 g de poudre de peau préchromée, puis filtre, évapore à siccité à l'aide d'un évaporateur rotatif 200 ml de filtrat et pèse le résidu, soit m en grammes.

Titre en tannin :

$$(1 - m) \times 100 \times \frac{100}{100-h}$$

5 - 6 - Dosage biologique :

Déterminé selon la méthode de SHINOGI (J. Pharm. Sci., 1985, 74 (4), 482-485 ) la dose de principe actif produisant une activité inhibitrice de 50 % (I 50) vis-à-vis de l'élastase pancréatique porcine avec comme substrat le tertio-butylcarbonylalanine p-nitrophénol (Boc-Ala-ONp) devra être de :

$$1 \ \mu g \pm 15 \ \%$$

EXEMPLE 6 - ACTIVITE BIOLOGIQUE DU PRINCIPE ACTIF

Le principe actif ainsi préparé présente des activités inhibitrices très importantes, vis-à-vis notamment de diverses enzymes protéolytiques telles que les élastase, collagénase et hyaluronidase. Les résultats figurent dans le tableau suivant dans lequel les résultats sont exprimés en masse d'inhibiteur (en μg), soit rapportée à la drogue sèche d'origine, soit réelle pour les principes définis, produisant une inhibition de l'activité enzymatique de 50 % (I 50) dans les conditions des essais. Le signe ( - ) indique une activité inhibitrice nulle.

| I 50 ( µg ) | | | | | |
|---|---|---|---|---|---|
| Inhibiteur | Elastase | Collagénase | Hyaluronidase | Tripsine | α-Chymotrypsine |
| Reine des Prés fleur | 10 | 40 | 30 | 30 | 35 |
| Alchémille plante | 2500 | $8 \times 10^3$ | $10^4$ | $5 \times 10^3$ | $7,5 \times 10^3$ |
| Rhubarbe racine | 50 | 150 | 240 | 90 | 130 |
| Acide tannique | 50 | | | | |
| Acide gallique | 100 | | | | |
| Hamamélitanin | 600 | | | | |
| Gallate de propyle | - | | | | |
| Catéchine-épicatéchine | 35 | | | | |
| Ac. chloro-génique | - | | | | |
| Salicylate de méthyle | - | | | | |
| Ethoxazo-rutine | 250 | | | | |
| Rutoside | 150 | | | | |
| Hypéroside | 275 | | | | |
| Quercétine 4'-Glucoside | 250 | | | | |
| Quercétine | 55 | | | | |
| Morine | 60 | | | | |
| Principe tannique de Filipendula Ulmaria | 1 | 5 | 6 | 3,5 | 5 |

Il faut noter que ces activités sont très constantes suivant les lots de préparation.

<u>6 - 1</u> - Protocoles des mesures des activités anti-enzymatiques :

a) Activités inhibitrices de l'élastase, de la trypsine et de l'α-chymotrypsine :

Les dosages d'activités inhibitrices de l'élastase, de la trypsine et de l'α-chymotrypsine ont été réalisés selon la méthode de SHINOGI et al. (1), la quantité d'inhibiteur étant adaptée de manière à obtenir une inhibition voisine de 50 % (I50) après 30 minutes, les extraits étant généralement préparés à partir de 0,5 g de poudre (250) de plante sèche dans 20 ml d'éthanol à 50 % V/V, les principes définis en solution à 1 mg/ml d'éthanol à 50 % V/V.

(1) SHINOGI M., AGHA B.J., TSUJI K. and DIGENIS G.A. Assessment of antiprotease activity of some carbamate derivatives, <u>J. Pharm. Sci.</u>, 1985, <u>74</u> (4), 482-485.

b) Activités inhibitrices de la collagénase :

Les dosages d'activités inhibitrices de la collagénase ont été réalisés selon la méthode viscosimétrique de MaC CARTNEY et al. (2) à l'aide d'un viscosimètre de type OSTWALD à tube capillaire permettant une mesure du temps d'écoulement pour l'eau à 27°C d'environ 30 secondes.

Pour les mesures d'activités inhibitrices 2,5 ml de collagénase (Sigma type IV à 2 μg/ml) en solution dans le tampon 0,05 M Tris/HCl pH 7,4 contenant 5mM de $CaCl_2$ sont mis à incuber à 27°C pendant 10 minutes avec 100 μl d'inhibiteur en solution dans l'éthanol à 50 % V/V, la quantité d'inhibiteur étant adaptée, les extraits végétaux et les principes définis préparés comme précédemment. Le substrat (2,5 ml de collagène SIGMA type III à 0,1 % V/V dans le même tampon) également thermostaté est alors ajouté. Les mesures de temps d'écoulement sont effectuées au départ puis toutes les 15 minutes pendant une heure, soit "t" la mesure après une heure.

On peut opérer par rapport à un témoin contenant 100 μl d'éthanol à 50 % V/V sans inhibiteur, soit "to" la mesure après une heure, puis calculer la masse d'inhibiteur I50 en fonction du % d'inhibition obtenu dans les conditions de l'essai.

$$\% \text{ d'inhibition} : \frac{to - t}{to} \times 100$$

(2) Mac CARTNEY H.W., BATES S.R.E., BLUMSON N.L., NELSON D., JAMISON D. and ELMORE D.T. A recording viscometer for assaying mammlian collagenase, <u>Biochem. J.</u>, 1983, <u>213</u>, 275-278.

c) Activité inhibitrice de l'hyaluronidase :

Les dosages d'activités inhibitrices de l'hyaluronidase ont été réalisés selon la méthode de réduction turbidimétrique de l'USP XX (United States Pharmacopoea, 1980, <u>20</u>, 376-377).

Pour les méthodes d'activités inhibitrices, 1 ml de solution d'hyaluronidase (SIGMA III-P à 0,75 unités/ml) sont mis à incuber à 37°C pendant 10 minutes avec 100 μl d'inhibiteur en solution dans l'éthanol à 50 % V/V, la quantité d'inhibiteur étant adaptée et les préparations effectuées comme précédemment. Le substrat (1 ml de solution d'hyaluronate de potassium à 0,25 mg/ml) également thermostaté est alors ajouté. Après incubation pendant 30 minutes à 37°C, 8 ml de solution de sérum sont ajoutés et la densité optique lue au spectrophotomètre à 640 nm après 30 minutes à température ambiante, soit D par rapport à un blanc sans hyaluronate.

On peut opérer par rapport à un témoin contenant 100 μl d'éthanol à 50 % V/V sans inhibiteur, soit Do par rapport à un blanc sans hyaluronate, puis calculer la masse d'inhibiteur I50 d'après le % d'inhibition obtenu dans les conditions de l'essai

$$\% \text{ d'inhibition} : \frac{Do - D}{Do} \times 100.$$

d) Application :

Le principe actif tannique de la Reine des Prés présente,du fait de ses activités,un effet anti-vieillissement général sur les organismes vivants par restauration notamment de l'intégrité du tissu conjonctif.

Il s'agit donc d'un produit anti-vieillissement, agissant en tant que protecteur cellulaire vis-à-vis d'agressions très diverses par fixation membranaire, pariétale ou tissulaire et par inhibition des enzymes dégradant la trame des membranes, des parois et des tissus d'organes très divers.

Il s'ensuit des propriétés très variées utilisables industriellement, notamment en médecine humaine et vété-

rinaire, en pharmacie, en cosmétologie, en diététique et dans tous autres domaines d'application également protégés.

Ainsi, par exemple, sont en outre indiquées des applications telles que les suivantes :

– en cosmétologie : produit anti-vieillissement, améliore les propriétés toni-élastiques de la peau (anti-élastase, anti-collagénase) au niveau facial (anti-rides), corporel (raffermissement des seins, anti-cellulitique) ou capillaire (tonique du cuir chevelu, anti-chute) ; produit adoucissant.

– en dermatologie : produit antiinflammatoire, restaure les propriétés toni-élastiques de la peau, améliore la circulation (vergetures, lipodystrophies, couperose), anti-infectieux en tant que antibactérien, antifongique et antiviral (acnés, points noirs, surinfections), cicatrisant, etc., au niveau corporel, facial ou du cuir chevelu :

– en cardioangéiologie : comme protecteur vasculaire veineux (varices, hémorroïdes), rétinien, cérébral par voie générale ou locale, comme antiathéromateux et hypolipémiant, comme anti-angoreux ;

– en gastro-entérologie : comme antiinflammatoire et hémostatique (anti-ulcère), anti-infectieux et anti-diarrhétique ;

– en gynécologie : en usage local comme hémostatique utérin, anti-infectieux, contraceptif ;

– en hématologie : comme hémostatique par voie générale ou locale (hémophilie) ;

– en rhumatologie : comme antiinflammatoire (rhumatismes divers) en usage local ou per os ;

– en stomatologie : comme protecteur du tissu gingival, antihémorragique, anti-infectieux, anti-inflammatoire (parodontoses) en usage local ou per os ;

– en urologie et néphrologie : comme anti-prostatique ;

– comme anti-infectieux général ; antibactérien, antifongique, antiviral ;

– en diabétologie : diabète gras.

## EXEMPLE 7 - TOXICOLOGIE

Des essais d'innocuité effectués classiquement chez le rat ont montré la bonne tolérance générale du principe actif, aucune toxicité n'ayant été observée aux doses maximales administrables par voie orale ou par voie parentérale.

Des essais effectués chez des femmes volontaires ont également montré l'innocuité du produit en application locale.

Il faut signaler la non toxicité généralement reconnue de la Reine des Prés, qui figure d'une part, dans la liste de plantes en vente libre en dehors des officines et des herboristeries - Décret n° 79-480 du 15 juin 1979 (J.O. 22-6-1979), choisies notamment en raison de leur absence de toxicité et, d'autre part, dans la liste de 72 plantes médicinales pour tisanes (B.O. du 7-3-1985) élargie depuis à 112 plantes pour les spécialités pharaceutiques à base de plantes (B. O. n° 86/20 bis d'août 1986) pouvant bénéficier sous certaines conditions d'un dossier allégé de demande d'Autorisation de Mise sur le Marché.

## EXEMPLE 8 - FORMES GALENIQUES

8 - 1 - Comprimés dragéifiés entériques :

Principe tannique de Filipendula Ulmaria 0,010 g Excipient* q.s.p. un comprimé à enrobage entérique. Excipient* : lactose, silice, stéarate de magnésium, polyvidone, phtalate de diéthyle, saccharose, talo, acétophatalate de cellulose, gomme arabique, oxyde de titane, sesquioxyde de fer, cire blanche, cire de Carnauba Q.S.

8 - 2 - Soluté buvable : Ampoules de 2 ml.

```
Principe tannique de Filipendula Ulmaria      0,010 g
Excipient :
Alcool à 95^C                                  1    ml
Eau purifiée q.s.p.                            2    ml.
```

8 - 3 - Soluté injectable : Ampoules de 2 ml.

| | | |
|---|---|---|
| Principe tannique de <u>Filipendula Ulmaria</u> | 0,010 | g |
| Excipient : | | |
| Monopropylène-glycol | 1 | ml |
| Eau p.p.i. q.s.p. | 2 | ml |

8 - 4 - Crème :

| | | |
|---|---|---|
| Principe tannique de <u>Filipendula Ulmaria</u> | 0,5 | g |
| Excipient : | | |
| Mono et diglycérides de l'acide palmitique | 8 | g |
| Alcool cétylique | 2 | g |
| Alcool cétostéarylique | 1 | g |
| Cétostéarate de polyoxyéthylène | 1 | g |
| Monooléate de glycérol | 1 | g |
| 2-Octyl dodécanol | 8 | g |
| Triglycéride de l'acide caprylique | 3 | g |
| Huile de paraffine fluide | 3 | g |
| Acide polyacrylique réticulé | 0,3 | g |
| Triéthanolamine | 0,8 | g |
| Parfum, conservateur, eau purifiée q.s.p. | 100 | g |

8 - 5 - Gel :

| | | |
|---|---|---|
| Principe tannique de <u>Filipendula Ulmaria</u> | 0,5 | g |
| Excipient . | | |
| Acide polyacrylique réticulé | 0,6 | g |
| Triéthanolamine | 0,6 | g |
| Monopropylèneglycol | 45 | g |
| Parfum, conservateur, eau purifiée q.s.p. | 100 | g |

**Revendications**

1. Procédé d'obtention de principes tanniques purifiés d'origine végétale, caractérisé en ce qu'il comporte essentiellement les étapes suivantes :
a) l'adsorption du principe tannique actif, à partir d'une solution extractive de la matière première, sur un adsorbant polymère à fonctions amides ou équivalentes, et
b) la désorption sélective et quantitative du principe tannique actif fixé sur l'adsorbant, désorption à l'aide de bases ou de substances à propriétés basiques.
2. Procédé selon la revendication 1, caractérisé en ce que la solution est ensuite neutralisée par addition

d'acide ou de substances à propriétés acides pures ou en mélanges.

3. Procédé selon l'une des revendications précédentes, caractérisé en ce que, entre l'étape d'adsorption a) et l'étape de désorption b) des substances banales de la plante sont éliminées quantitativement par lavage ou élution à l'aide de solvants.

4. Procédé selon l'une des revendications précédentes, caractérisé en ce que la plante d'origine est la Reine des Prés - Filipendula Ulmaria (Flora Europaea) ou Spiraea Ulmaria - de la famille des Rosacées, l'espèce proprement dite, ses sous-espèces, ses variétés ou espèces affines, notamment d'origine horticole et les hybrides éventuels.

5. Procédé selon l'une des revendications précédentes, caractérisée en ce que les polymères d'adsorption à fonctions amides ou équivalentes sont choisis parmi notamment les nylons®, les polyamides, les polyvinylpyrrolidones, soit purs, soit mélangés entre eux ou dilués.

6. Procédé selon l'une des revendications précédentes, caractérisé en ce que la désorption se fait avec des bases ou des substances à propriétés basiques, fortes ou faibles, en solutions aqueuses, alcooliques, ou cétoniques, pures ou en mélanges.

7. Procédé selon l'une des revendications précédentes, caractérisé en ce que la substance à propriétés basiques réalisant la désorption est une solution d'hydroxyde de sodium ou une solution d'ammoniaque.

8. Procédé selon l'une des revendications précédentes, caractérisé en ce que la neutralisation de la solution après l'étape b), neutralisation par addition d'acides ou substances à propriétés acides peut se faire par utilisation de résines échangeuses d'ions, par dialyse.

9. Procédé selon l'une des revendications précédentes, caractérisé en ce que l'extraction a lieu à partir de la drogue fraîche ou desséchée divisée de manière suffisante sous forme de poudre selon un quelconque procédé, l'extraction a lieu à l'aide de solvants d'extraction et est effectuée suivant un procédé à chaud ou à froid avec un appareillage d'extraction de quelconque nature opérant en continu ou en discontinu.

10. Procédé selon la revendication 9, caractérisé en ce qu'une extraction préliminaire, de manière à éliminer des substances banales gênant l'extraction, peut être réalisée par exemple avec un solvant de polarité différente de celui de l'extraction.

11. Procédé selon les revendications 9 et 10, caractérisé en ce que, après extraction et avant purification, les parties végétales peuvent être séparées de la solution extractive à l'aide d'un appareillage adapté par exemple de filtration ou de centrifugation et, enfin, la solution extractive peut être concentrée ou évaporée à siccité et reprise par des solvants.

12. Procédé selon l'une des revendications précédentes, caractérisé en ce que les solvants, notamment d'extraction ou de lavage, sont choisis parmi :

. les solvants hydroxylés par exemple l'eau, les solutions aqueuses salines, acides ou basiques, les alcools, notamment le méthanol, l'éthanol, le propanol ou encore le propylèneglycol, le glycérol,

. les solvants cétoniques, notamment l'acétone, le méthyléthylcétone,

. les esters tels que acétate d'éthyle, acétate d'isopropyle,

. ou tout autre solvant à caractère relativement polaire, tels que le tétrahydrofuranne,

ces solvants pouvant être utilisés soit purs, soit mélangés entre eux en quelconques proportions.

13. Procédé selon l'une des revendications précédentes, caractérisé en ce que les liqueurs de désorption ainsi obtenues peuvent être ensuite traitées de manière à obtenir le produit actif sous forme sèche, par exemple les liqueurs de désorption obtenues peuvent être concentrées de manière telle que le produit actif précipite de la solution, la précipitation étant rendue quantitative par exemple par refroidissement ou tout autre procédé permettant de quantifier cette opération, et le produit actif peut être récupéré suivant une opération de séparation, telle que par exemple une centrifugation, une filtration ou tout autre type d'opérations permettant l'élimination du liquide et la récupération du produit actif sous forme solide, le principe actif étant ensuite soumis à une opération de dessiccation de quelconque nature, de manière à obtenir un produit suffisamment sec pour pouvoir être réduit en poudre.

14. Procédé selon l'une des revendications précédentes, caractérisé en ce que la solution extractive est extraite de poudre, de fleurs de Reine des Prés, puis elle est filtrée, puis concentrée jusqu'à élimination du solvant, le résidu est ensuite repris avec de l'eau, soumis à une température supérieure à la température ambiante de manière à faciliter la dissolution, la solution aqueuse obtenue est déposée en tête d'une colonne contenant de la poudre de polymère polyamidé, après adsorption de la solution aqueuse, la colonne est lavée avec un solvant, la matière banale étant ainsi éliminée, l'élution est ensuite réalisée à l'aide de solutions hydroalcooliques et d'hydroxyde de sodium ou d'ammoniaque, l'éluat étant neutralisée en cours d'élution avec de l'acide chlorhydrique ou de l'acide acétique, la solution est ensuite soumise à la dialyse, puis la liqueur de dialyse est concentrée jusqu'à apparition d'un précipité par exemple à l'aide d'un évaporateur rotatif sous pression réduite, la liqueur concentrée est ensuite réfrigérée à 4°C de manière à quantifier la précipitation, la liqueur est filtrée sur un Büchner, le précipité est lavé et séché, le produit sec est alors pulvérisé.

15. Composition à activité anti-enzymatique caractérisée en ce qu'elle comporte le principe actif sous forme sèche ou liquide obtenu par le procédé selon l'une des revendications précédentes.

16. Composition selon la revendication 15 caractérisée en ce qu'elle a une activité anti-élastase, anti-trypsine, anti-$\alpha$-chymotrypsine, anti-collagénase ou anti-hyalurodinase.

17. Utilisation en cosmétologie d'une composition selon les revendications 15 ou 16.

18. Médicament contenant une composition selon les revendications 15 ou 16.

## Claims

1. A process for obtaining purified tanning agents of vegetable origin, characterized in that it comprises essentially the following steps:

   a) the adsorption of the active tanning agent from an extractive solution of the raw material on a polymeric adsorbent with amide or equivalent functions, and

   b) the selective and quantitative desorption, with the aid of bases or of substances with basic properties, of the active tanning agent fixed on the adsorbent.

2. A process according to claim 1, characterized in that the solution is then neutralized by the addition of acid or of pure or mixed substances with acidic properties.

3. A process according to one of the preceding claims, characterized in that between the adsorption step a) and the desorption step b), commonplace plant substances are quantitatively eliminated by washing or elution with the aid of solvents.

4. A process according to one of the preceding claims, characterized in that the plant of origin is the meadowsweet - Filipendula Ulmaria (Flora Europaea) or Spiraea Ulmaria - of the Rosaceae family, the species properly so-called, its subspecies, its varieties or related species, especially of horticultural origin, and possible hybrids.

5. A process according to one of the preceding claims, characterized in that the adsorbent polymers with amide or equivalent functions are chosen especially from the nylons®, the polyamides and the polyvinylpyrrolidones, either pure or mixed with each other or diluted.

6. A process according to one of the preceding claims, characterized in that the desorption occurs with bases or substances with basic properties, strong or weak, in aqueous, alcoholic or ketonic solutions, pure or in mixtures.

7. A process according to one of the preceding claims, characterized in that the substance with basic properties bringing about the desorption is a sodium hydroxide or ammonia solution.

8. A process according to one of the preceding claims, characterized in that the neutralization, by addition of acids or substances with acidic properties, of the solution after step b), can occur by use of ion exchanger resins or by dialysis.

9. A process according to one of the preceding claims, characterized in that the extraction is carried out on the fresh or dried-out pharmaceutical raw material, divided sufficiently in the form of a powder by any process, and the extraction is carried out with the aid of extraction solvents and by a process in the hot or in the cold with an extraction apparatus of any kind, operating continuously or discontinuously.

10. A process according to claim 9, characterized in that a preliminary extraction, so as to eliminate the commonplace substances interfering with the extraction, can be carried out for example with a solvent of polarity different from the extraction solvent.

11. A process according to claims 9 and 10, characterized in that, after extraction and before purification, the vegetable components can be separated from the extractive solution with the aid of a suitable apparatus, for example for filtration or centrifugation, and finally the extractive solution can be concentrated or evaporated to dryness and taken up in solvents.

12. A process according to one of the preceding claims, characterized in that the solvents, especially for extraction or washing, are chosen from:

   . hydroxylated solvents, for example water, acidic or basic aqueous saline solutions, alcohols, especially methanol, ethanol, propanol or also propylene glycol or glycerol.

   . ketonic solvents, especially acetone and methyl ethyl ketone,

   . esters such as ethyl acetate and isopropyl acetate,

   . or any other solvent of relatively polar character, such as tetrahydrofuran,

   these solvents possibly being used either pure or mixed with each other in any proportions.

13. A process according to one of the preceding claims, characterized in that the desorption solutions thus obtained can then be treated so as to obtain the active product in dry form, for example the desorption solutions obtained can be concentrated so that the active product precipitates from solution, the precipitation being made quantitative, for example by cooling or any other process enabling this operation to be made quantitative, and the active product can be recovered by a separation operation, such as for example a centrifugation, a filtration or any other the of operation enabling the liquid to be eliminated and the active product to be recovered in solid form, the active agent then being subjected to any kind of desiccation so as to obtain a product sufficiently dry to enable it to be reduced to powder.

14. A process according to one of the preceding claims, characterized in that the extractive solution is extracted from powdered meadowsweet flowers, then filtered and then concentrated until the solvent is eliminated, the residue is then taken up in water, subjected to a temperature above the ambient so as to facilitate dissolution, the aqueous solution obtained is deposited at the head of a column containing powdered polyamide polymer, after adsorption of the aqueous solution the column is washed with a solvent, the commonplace material thus being eliminated, the elution is then carried out with aqueous alcoholic solutions and sodium or ammonium hydroxide solutions, the eluate being neutralized during elution with hydrochloric acid or acetic acid, the solution is then subjected to dialysis, the dialyzate solution is then concentrated, for example with the aid of a rotary evaporator under reduced pressure, until a precipitate appears, the concentrated solution is then refrigerated at 4 °C so as to make the precipitation quantitative, the solution is filtered on a Büchner filter, the precipitate is washed and dried, and the dry product is then ground.

15. A composition with anti-enzymatic activity, characterized in that it contains the active agent in the dry or liquid form obtained by the process according to one of the preceding claims.

16. A composition according to claim 15, characterized in that it has an anti-elastase, anti-trypsin, anti-$\alpha$-chymotrypsin, anti-collagenase or anti-hyalurodinase activity.

17. The use in cosmetology of a composition according to claims 15 or 16.

18. A medicament containing a composition according to claims 15 or 16.

## Patentansprüche

1. Verfahren zur Herstellung von gereinigten Gerbstoffen pflanzlichen Ursprungs, **dadurch gekennzeichnet,** daß es im wesentlichen folgende Stufen umfaßt:

a) die Adsorption der aktiven Gerbstoffe aus einer Extraktionslösung des Ausgangsmaterials an einen polymeren Adsorbens mit Amidfunktionen oder Äquivalenten, und

b) die selektive und quantitative Desorption der an das Adsorbens fixierten aktiven Gerbstoffe, wobei die Desorption mit Basen oder mit Substanzen mit basischen Eigenschaften erfolgt.

2. Verfahren nach Anspruch 1, dadurch gekennzeichnet, daß die Lösung anschließend durch Zusatz von Säure oder von Substanzen mit sauren Eigenschaften, allein oder im Gemisch, neutralisiert wird.

3. Verfahren nach einem der vorhergehenden Ansprüche, dadurch gekennzeichnet, daß zwischen der Adsorptionsstufe a) und der Desorptionsstufe b) die gewöhnlichen Substanzen der Pflanze quantitativ durch Wäsche oder Eluieren mit Lösungsmitteln entfernt werden.

4. Verfahren nach einem der vorhergehenden Ansprüche, dadurch gekennzeichnet, daß die Ursprungspflanze Geißbart - Filipendula Ulmaria (Flora Europaea) oder Spiraea Ulmaria - aus der Familie der Rasaceen, die eigentliche Spezies, ihre Subspezies, ihre Arten oder verwandten Spezies, insbesondere aus dem Gartenbau und mögliche Hybriden, sind.

5. Verfahren nach einem der vorhergehenden Ansprüche, dadurch gekennzeichnet, daß die polymeren zur Adsorption mit Amidfunktionen oder Äquivalenten ausgewählt werden aus den Nylons[R], den polyamiden, den Polyvinylpyrrolidonen, entweder allein oder im Gemisch miteinander oder verdünnt.

6. Verfahren nach einem der vorhergehenden Ansprüche, dadurch gekennzeichnet, daß die Desorption mit starken oder schwachen Basen oder Substanzen mit basischen Eigenschaften, in wässriger, alkoholischer oder ketonischer Lösung allein oder im Gemisch, durchgeführt wird.

7. Verfahren nach einem der vorhergehenden Ansprüche, dadurch gekennzeichnet, daß die Substanz mit basischen Eigenschaften zur Durchführung der Desorption eine Natriumhydroxydlösung oder eine Ammoniaklösung ist.

8. Verfahren nach einem der vorhergehenden Ansprüche, dadurch gekennzeichnet, daß die Neutralisation der Lösung nach der Stufe b) durch Zusatz von Säuren oder Substanzen mit sauren Eigenschaften durch Verwendung von Ionenaustauscherharzen, durch Dialyse, erfolgen kann.

9. Verfahren nach einem der vorhergehenden Ansprüche, dadurch gekennzeichnet, daß die Extraktion an der frischen oder getrockneten, in ausreichender Weise nach beliebigen Verfahren zur pulverform zerkleinerten Droge erfolgt, wobei die Extraktion mit Extraktionslösungsmitteln durchgeführt wird und nach einem Verfahren in der Wärme oder in der Kälte mit einer beliebigen Extraktionsvorrichtung, die kontinuierlich oder diskontinuierlich arbeitet, durchgeführt wird.

10. Verfahren nach Anspruch 9, dadurch gekennzeichnet, daß eine erste Extraktion zur Entfernung der gewöhnlichen, die Extraktion störenden Substanzen durchgeführt werden kann, beispielsweise mit einem Lösungsmittel, dessen Polarität von dem zur Extraktion verschieden ist.

11. Verfahren nach Anspruch 9 und 10, dadurch gekennzeichnet, daß nach der Extraktion und vor der Reinigung die pflanzlichen Teile aus der Extraktionslösung mit einer geeigneten Vorrichtung, beispielsweise zur Filtration oder zum Zentrifugieren, abgetrennt werden können und anschließend die Extraktionslösung zur Trockne konzentriert oder verdampft werden kann und mit Lösungsmitteln aufgenommen wird.

12. Verfahren nach einem der vorhergehenden Ansprüche, dadurch gekennzeichnet, daß die Lösungsmittel insbesondere zur Extraktion und zur Wäsche ausgewählt werden aus:

. hydroxylierten Lösungsmitteln, beispielsweise Wasser, wässrigen salzhaltigen, sauren oder basischen Lösungen, Alkoholen, insbesondere Methanol, Ethanol, Propanol oder auch Propylenglykol, Glycerin,

. den Ketonlösungsmitteln, insbesondere Aceton, Methylethylketon,

. den Estern, wie Ethylacetat, Isopropylacetat,

. oder jedem anderen Lösungsmittel mit relativ polarem Charakter, wie Tetrahydrofuran,

wobei diese Lösungsmittel entweder allein oder im Gemisch miteinander in jeglichen Anteilen verwendet werden können.

13. Verfahren nach einem der vorhergehenden Ansprüche, dadurch gekennzeichnet, daß die so erhaltenen Desorptionsflüssigkeiten anschließend behandelt werden können, um das aktive Produkt in trockener Form zu erhalten, wobei beispielsweise die erhaltenen Desorptionsflüssigkeiten so konzentriert werden können, daß das aktive Produkt aus der Lösung ausfällt, wobei die Ausfällung quantitativ wird beispielsweise durch Abkühlen oder nach jeglichem anderen Verfahren , das dazu geeignet ist, dieses Verfahren quantitativ zu gestalten, und das aktive Produkt mittels eines Trennverfahrens, wie beispielsweise Zentrifugieren, Filtrieren oder jeglicher andere Verfahrenstyp, der die Entfernung von Flüssigkeit und die Gewinnung des aktiven Produkts in fester Form ermöglicht, gewonnen werden kann, wobei das aktive Prinzip anschließend einer Trocknung jeglicher Art so unterzogen wird, daß ein ausreichend trockenes Produkt zur Zerkleinerung zu einem Pulver erzielt wird.

14. Verfahren nach einem der vorhergehenden Ansprüche, dadurch gekennzeichnet, daß die Extraktionslösung vom pulver von Geißblatt abgezogen, anschließend filtriert und anschließend konzentriert wird bis zur Entfernung des Lösungsmittels, worauf der Rückstand wieder mit Wasser aufgenommen wird, auf eine Temperatur über der Umgebungstemperatur zur Erleichterung der Auflösung erwärmt wird, die erhaltene wässrige Lösung in den Kopf einer Kolonne eingebracht wird, die das Pulver des Polyamidpolymeren enthält, die Kolonne nach der Adsorption der wässrigen Lösung mit einem Lösungsmittel derart gewaschen wird, daß das gewöhnliche Material entfernt wird, worauf mit wässrig-alkoholischen Lösungen und Natriumhydroxyd oder Ammoniak eluiert wird, das Eluat im Verlauf des Eluierens mit Chlorwasserstoffsäure oder Essigsäure neutralisiert wird, die Lösung dann einer Dialyse unterworfen wird, worauf die Dialyseflüssigkeit bis zum Auftreten einer Ausfällung, beispielsweise mittels eines Rotationsverdampfers unter verringertem Druck, konzentriert wird, die konzentrierte Flüssigkeit anschließend auf 4°C abgekühlt wird, um die Ausfällung quantitativ zu machen, die Flüssigkeit über einen Büchner-Trichter filtriert wird, die Ausfällung gewaschen und getrocknet wird und das trockene Produkt dann pulverisiert wird.

15. Zusammensetzung mit antienzymatischer Aktivität, dadurch gekennzeichnet, daß sie das aktive Prinzip in trockener oder flüssiger Form, erhalten nach dem Verfahren eines der vorhergehenden Ansprüche, enthält.

16. Zusammensetzung nach Anspruch 15, dadurch gekennzeichnet, daß sie eine Anti-Elastase-, Anti-Trypsin-, Anti-$\alpha$-Chymotrypsin-, Anti-Kollagenase- oder Anti-Hyalurodinase-Aktivität hat.

17. Verwendung einer Zusammensetzung gemäß den Ansprüchen 15 oder 16 in der Kosmetik.

18. Arzneimittel, enthaltend eine Zusammensetzung nach einem der Ansprüche 15 oder 16.